# EUROPEAN PATENT SPECIFICATION

(11) **EP 3 524 168 B1**
(45) Date of publication and mention of the grant of the patent: **04.08.2021**
(21) Application number: 18200566.0
(22) Date of filing: 16.10.2018
(51) Int. Cl.: A61B 8/08, A61B 8/00

(54) **ULTRASOUND IMAGING APPARATUS AND METHOD OF CONTROLLING SAME**
ULTRASCHALLBILDGEBUNGSVORRICHTUNG UND VERFAHREN ZUR STEUERUNG DAVON
APPAREIL D'IMAGERIE À ULTRASONS ET PROCÉDÉ DE COMMANDE CORRESPONDANT

(30) Priority: 13.02.2018 KR 20180018062
(43) Date of publication of application: 14.08.2019
(73) Proprietor: Samsung Medison Co., Ltd., Hongcheon-gun, Gangwon-do, 25108 (KR)
(72) Inventor: KIM, Jung-ho, Gyeonggi-do (KR); LEE, Yoon-chang, Gyeonggi-do (KR)
(74) Representative: Grootscholten, Johannes A.M.

(56) References cited:
- KR-A- 20170 053 103
- US-A1- 2002 165 454
- US-A1- 2009 112 097
- US-B1- 6 319 204

## Description

### 1. Field

The disclosure relates to an ultrasound imaging apparatus and a method of controlling the ultrasound imaging apparatus.

### 2. Description of Related Art

Ultrasound diagnostic apparatuses transmit, to an object, ultrasound signals generated by transducers of a probe and detect information about signals reflected from the object, thereby obtaining at least one image of an internal part, for example, soft tissue or blood flow, of the object.

In addition, ultrasound diagnosis apparatuses utilize ultrasound contrast agents made of micro-bubbles to obtain ultrasound contrast agent images for diagnosis of various lesions. The ultrasound contrast agents made of micro-bubbles act as reflectors that are robust against ultrasound signals. Therefore, since echo signals reflected from the ultrasound contrast agents are stronger than other general echo signals, information about blood vessels into which the contrast agents are injected may be obtained in more detail through the ultrasound contrast agent images.

Here, reference is made to prior-art document US 6 319 204 B1, which is considered to constitute the closest known prior-art. The present invention may be distinguished from the teachings of US 6 319 204 B1 at least by the generation of n cumulative ultrasound contrast images; and the display of the generated n cumulative ultrasound contrast images sequentially in the order of time elapse, in accordance with the appended independent claims.

### SUMMARY

In accordance with one aspect of the present invention, there are provided are ultrasound imaging apparatuses exhibiting the features specified in the appended first and second independent apparatus claims.

In accordance with another aspect of the present invention, there is provided a method of controlling an ultrasound imaging apparatus including the steps specified in the appended independent method claim .

In accordance with another aspect of the disclosure, a method of controlling an ultrasound imaging apparatus includes: acquiring a plurality of ultrasound contrast agent images for a region of interest of the object after an ultrasound signal for destroying a contrast agent is transmitted to the region of interest of the object, into which the contrast agent is injected, at time intervals of tₖ (k 1, 2, ..., n, and n is an integer of 2 or more), generating a k^{th} cumulative ultrasound contrast agent image by accumulating the plurality of acquired ultrasound contrast agent images at every time intervals of tₖ to generate n cumulative ultrasound contrast agent images; and displaying the generated n cumulative ultrasound contrast agent images, wherein tₖ satisfies tₖ > tₖ₋₁.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is a block diagram illustrating an ultrasound diagnosis apparatus according to an exemplary embodiment;
FIGS. 2A, 2B, and 2C are diagrams respectively illustrating an ultrasound diagnosis apparatus according to an exemplary embodiment;
FIG. 3 is a block diagram illustrating an ultrasound imaging apparatus according to an embodiment;
FIG. 4 is a flowchart of a method by which an ultrasound imaging apparatus displays a cumulative ultrasound contrast agent image of a region of interest of an object, according to an embodiment;
FIG. 5 is a flowchart of a method by which an ultrasound imaging apparatus generates and displays a cumulative ultrasound contrast agent image of a region of interest of an object, according to an embodiment;
FIG. 6 is a diagram illustrating an example of an ultrasound signal transmitted to an object by an ultrasound imaging apparatus, according to an embodiment;
FIG. 7 is a diagram illustrating an example in which the number of ultrasound contrast agent bubbles of an object is changed as an ultrasound imaging apparatus transmits an ultrasound signal to an object;
FIG. 8 is a diagram illustrating an example in which an ultrasound imaging apparatus displays a cumulative ultrasound contrast agent image of a region of interest of an object, according to an embodiment; and
FIG. 9 is a diagram illustrating an example of a video edited so that an ultrasound imaging apparatus sequentially reproduces a plurality of cumulative ultrasound contrast agent images of a region of interest of an object, according to an embodiment.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items.

Certain exemplary embodiments are described in greater detail below with reference to the accompanying drawings.

In the following description, the same drawing reference numerals are used for the same elements even in different drawings. The matters defined in the description, such as detailed construction and elements, are provided to assist in a comprehensive understanding of exemplary embodiments. However, it is to be understood that exemplary embodiments can be carried out without such specifically defined matters. Also, well-known functions or constructions are not described in detail since they may obscure exemplary embodiments with unnecessary detail.

Terms such as "part" and "portion" used herein denote entities that may be embodied by software or hardware. According to exemplary embodiments, a plurality of parts or portions may be embodied by a single unit or element, or a single part or portion may include a plurality of elements.

In exemplary embodiments, an image may include any medical image acquired by various medical imaging apparatuses such as a magnetic resonance imaging (MRI) apparatus, a computed tomography (CT) apparatus, an ultrasound imaging apparatus, or an X-ray apparatus.

Also, in the present specification, an "object", which is a thing to be imaged, may include a human, an animal, or a part thereof. For example, an object may include a part of a human, that is, an organ or a tissue, or a phantom.

Throughout the specification, an ultrasound image refers to an image of an object processed based on ultrasound signals transmitted to the object and reflected therefrom.

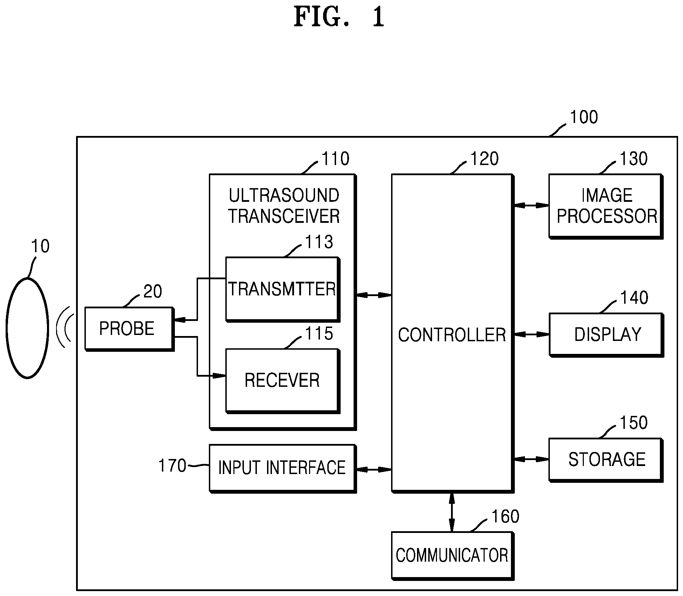

FIG. 1 is a block diagram illustrating a configuration of an ultrasound diagnosis apparatus 100, i.e., a diagnostic apparatus, according to an exemplary embodiment.

Referring to FIG. 1, the ultrasound diagnosis apparatus 100 may include a probe 20, an ultrasound transceiver 110, a controller 120, an image processor 130, one or more displays 140, a storage 150, e.g., a memory, a communicator 160, i.e., a communication device or an interface, and an input interface 170.

The ultrasound diagnosis apparatus 100 may be of a cart-type or a portable-type ultrasound diagnosis apparatus, that is portable, moveable, mobile, or hand-held. Examples of the portable-type ultrasound diagnosis apparatus 100 may include a smart phone, a laptop computer, a personal digital assistant (PDA), and a tablet personal computer (PC), each of which may include a probe and a software application, but embodiments are not limited thereto.

The probe 20 may include a plurality of transducers. The plurality of transducers may transmit ultrasound signals to an object 10 in response to transmitting signals received by the probe 20, from a transmitter 113. The plurality of transducers may receive ultrasound signals reflected from the object 10 to generate reception signals. In addition, the probe 20 and the ultrasound diagnosis apparatus 100 may be formed in one body (e.g., arranged in a single housing), or the probe 20 and the ultrasound diagnosis apparatus 100 may be formed separately (e.g., arranged separately in separate housings) but linked by wire or wirelessly. In addition, the ultrasound diagnosis apparatus 100 may include one or more probes 20 according to embodiments.

The controller 120 may control the transmitter 113 for the transmitter 113 to generate transmitting signals to be applied to each of the plurality of transducers based on a position and a focal point of the plurality of transducers included in the probe 20.

The controller 120 may control the ultrasound receiver 115 to generate ultrasound data by converting reception signals received from the probe 20 from analogue to digital signals and summing the reception signals converted into digital form, based on a position and a focal point of the plurality of transducers.

The image processor 130 may generate an ultrasound image by using ultrasound data generated from the ultrasound receiver 115.

The display 140 may display a generated ultrasound image and various pieces of information processed by the ultrasound diagnosis apparatus 100. The ultrasound diagnosis apparatus 100 may include two or more displays 140 according to the present exemplary embodiment. The display 140 may include a touch screen in combination with a touch panel.

The controller 120 may control the operations of the ultrasound diagnosis apparatus 100 and flow of signals between the internal elements of the ultrasound diagnosis apparatus 100. The controller 120 may include a memory for storing a program or data to perform functions of the ultrasound diagnosis apparatus 100 and a processor and/or a microprocessor (not shown) for processing the program or data. For example, the controller 120 may control the operation of the ultrasound diagnosis apparatus 100 by receiving a control signal from the input interface 170 or an external apparatus.

The ultrasound diagnosis apparatus 100 may include the communicator 160 and may be connected to external apparatuses, for example, servers, medical apparatuses, and portable devices such as smart phones, tablet personal computers (PCs), wearable devices, etc., via the communicator 160.

The communicator 160 may include at least one element capable of communicating with the external apparatuses. For example, the communicator 160 may include at least one among a short-range communication module, a wired communication module, and a wireless communication module.

The communicator 160 may receive a control signal and data from an external apparatus and transmit the received control signal to the controller 120 so that the controller 120 may control the ultrasound diagnosis apparatus 100 in response to the received control signal.

The controller 120 may transmit a control signal to the external apparatus via the communicator 160 so that the external apparatus may be controlled in response to the control signal of the controller 120.

For example, the external apparatus connected to the ultrasound diagnosis apparatus 100 may process the data of the external apparatus in response to the control signal of the controller 120 received via the communicator 160.

A program for controlling the ultrasound diagnosis apparatus 100 may be installed in the external apparatus. The program may include instructions to perform part of an operation of the controller 120 or the entire operation of the controller 120.

The program may be pre-installed in the external apparatus or may be installed by a user of the external apparatus by downloading the program from a server that provides applications. The server that provides applications may include a recording medium where the program is stored.

The storage 150 may store various data or programs for driving and controlling the ultrasound diagnosis apparatus 100, input and/or output ultrasound data, ultrasound images, applications, etc.

The input interface 170 may receive a user's input to control the ultrasound diagnosis apparatus 100 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an exemplary embodiment is not limited thereto.

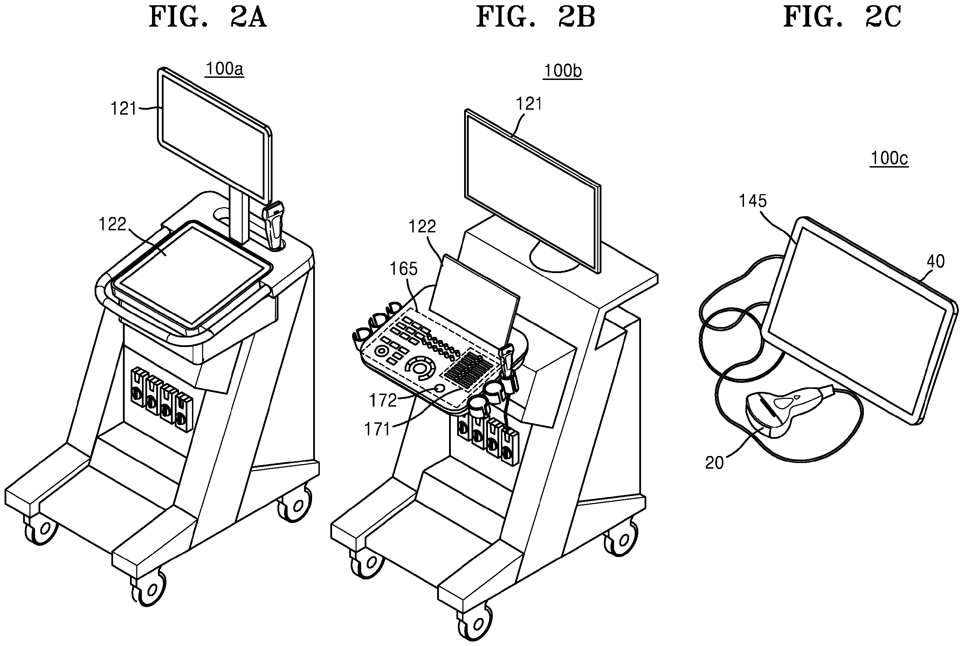

An example of the ultrasound diagnosis apparatus 100 according to the present exemplary embodiment is described below with reference to FIGS. 2A, 2B, and 2C.

FIGS. 2A, 2B, and 2C are diagrams respectively illustrating an ultrasound diagnosis apparatus according to an exemplary embodiment.

Referring to FIGS. 2A and 2B, the ultrasound diagnosis apparatus 100 may include a main display 121 and a sub-display 122. At least one among the main display 121 and the sub-display 122 may include a touch screen. The main display 121 and the sub-display 122 may display ultrasound images and/or various information processed by the ultrasound diagnosis apparatus 100. The main display 121 and the sub-display 122 may provide graphical user interfaces (GUIs), thereby receiving a user's inputs of data to control the ultrasound diagnosis apparatus 100. For example, the main display 121 may display an ultrasound image and the sub-display 122 may display a control panel to control display of the ultrasound image as a GUI. The sub-display 122 may receive an input of data to control the display of an image through the control panel displayed as a GUI. The ultrasound diagnosis apparatus 100 may control the display of the ultrasound image on the main display 121 by using the input control data.

Referring to FIG. 2B, the ultrasound diagnosis apparatus 100 may include a control panel 165. The control panel 165 may include buttons, trackballs, jog switches, or knobs, and may receive data to control the ultrasound diagnosis apparatus 100 from the user. For example, the control panel 165 may include a time gain compensation (TGC) button 171 and a freeze button 172. The TGC button 171 is to set a TGC value for each depth of an ultrasound image. Also, when an input of the freeze button 172 is detected during scanning an ultrasound image, the ultrasound diagnosis apparatus 100 may keep displaying a frame image at that time point.

The buttons, trackballs, jog switches, and knobs included in the control panel 165 may be provided as a GUI to the main display 121 or the sub-display 122.

Referring to FIG. 2C, the ultrasound diagnosis apparatus 100 may include a portable device. An example of the portable ultrasound diagnosis apparatus 100 may include, for example, smart phones including probes and applications, laptop computers, personal digital assistants (PDAs), or tablet PCs, but an exemplary embodiment is not limited thereto.

The ultrasound diagnosis apparatus 100 may include the probe 20 and a main body 40. The probe 20 may be connected to one side of the main body 40 by wire or wirelessly. The main body 40 may include a touch screen 145. The touch screen 145 may display an ultrasound image, various pieces of information processed by the ultrasound diagnosis apparatus 100, and a GUI.

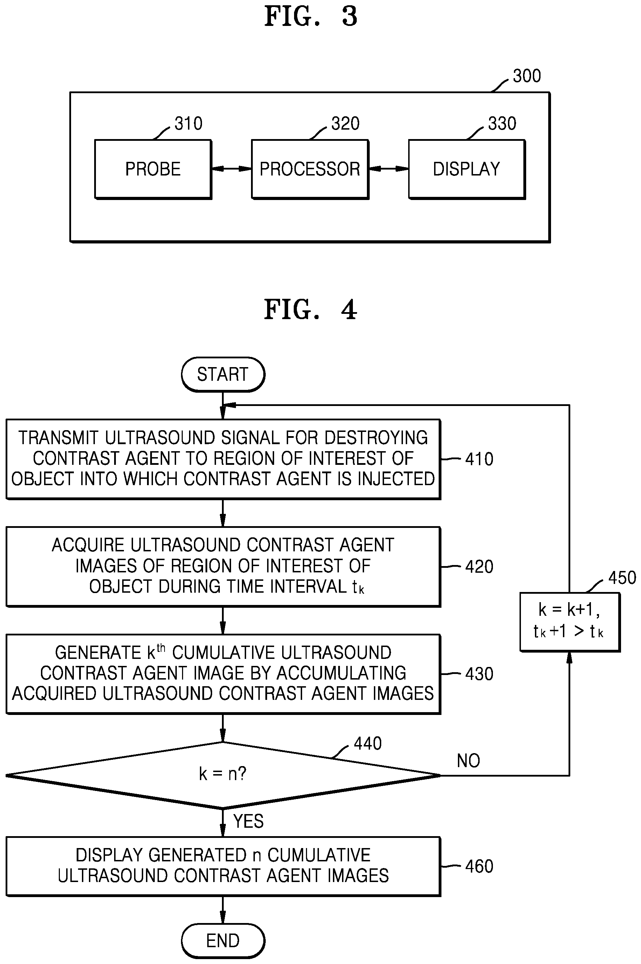

FIG. 3 is a block diagram illustrating an ultrasound imaging apparatus 300 according to an embodiment. The ultrasound imaging apparatus 300 may correspond to the ultrasound diagnosis apparatus 100 of FIG. 1. In addition, the ultrasound imaging apparatus 300 may be implemented in the form of the ultrasound diagnosis apparatuses 100a, 100b, and 100c.

As illustrated in FIG. 3, the ultrasound imaging apparatus 300 may include a probe 310, a processor 320, and a display 330. The probe 310 may correspond to the probe 20 of FIG. 1. The processor 320 may correspond to the controller 120 and the image processor 130 of FIG. 1. The display 330 may correspond to the display 140 of FIG. 1. In addition, the processor 320 may include one or more processors.

The probe 310 may transmit an ultrasound signal to an object and detect an echo signal. According to an embodiment, the probe 310 may transmit an ultrasound signal to a region of interest of an object into which a contrast agent is injected, and detect a contrast agent signal reflected from the contrast agent as an echo signal. For example, the probe 310 may transmit an ultrasound signal to a region of interest of an object while a contrast agent is injected into the region of interest of the object and the contrast agent is rising, and may detect, as an echo signal, a contrast agent signal for the rising contrast agent and a contrast agent signal for a state in which the contrast agent has risen.

According to an embodiment, the ultrasound signal transmitted to the object by the probe 310 so as to detect the echo signal may be an ultrasound signal of a sound pressure having an energy level that does not destroy the contrast agent. Therefore, the contrast agent injected into the object may not be destroyed by the ultrasound signal transmitted to the object by the probe 310 so as to detect the echo signal.

The probe 310 may transmit an ultrasound signal for destroying the contrast agent to the object. According to an embodiment, the probe 310 may destroy the contrast agent by transmitting an ultrasound signal of a sound pressure having an energy level for destroying the contrast agent to the region of interest of the object into which the contrast agent is injected. For example, the probe 310 may destroy the contrast agent by transmitting the ultrasound signal having a high sound pressure for destroying the contrast agent.

According to an embodiment, the probe 310 may transmit an ultrasound signal to a region of interest of an object while a contrast agent is rising again in the region of interest of the object after the contrast agent is destroyed, and may detect a contrast agent signal for the rising contrast agent as an echo signal. When the contrast agent is destroyed by transmitting the ultrasound signal having a high sound pressure to the region of interest of the object in which the contrast agent has risen, the contrast agent rises again in the region of interest of the object for a predetermined time. Therefore, the probe 310 may detect, as an echo signal, a plurality of contrast agent signals when the contrast agent rises in the region of interest of the object by transmitting the ultrasound signal to the object for a predetermined time after the contrast agent is destroyed.

The processor 320 may control the probe 310 to destroy the contrast agent by transmitting the ultrasound signal for destroying the contrast agent to the region of interest of the object into which the contrast agent is injected, and to detect a plurality of contrast agent signals.

According to an embodiment, the processor 320 may control the probe 310 to detect a plurality of contrast agent signals by transmitting an ultrasound signal for detecting the plurality of contrast agent signals to a region of interest of an object in which the contrast agent is rising again after an ultrasound signal for destroying the contrast agent is transmitted to the region of interest of the object, into which the contrast agent is injected, at time intervals of tₖ. In other words, the processor 320 may control the probe 310 to perform the process of detecting the plurality of contrast agent signals n times from k=1 to k=n during the time interval of tₖ after the contrast agent is destroyed.

According to an embodiment, the processor 320 may control the probe 310 to detect fₖ contrast agent signals by transmitting an ultrasound signal for detecting the plurality of contrast agent signals to a region of interest of an object in which the contrast agent is rising again after an ultrasound signal for destroying the contrast agent is transmitted to the region of interest of the object into which the contrast agent is injected. In other words, the processor 320 may control the probe 310 to perform the process of detecting fₖ contrast agent signals n times from k=1 to k=n after the contrast agent is destroyed.

The processor 320 may set the time interval of tₖ to detect the plurality of contrast agent signals after the contrast agent is destroyed. According to an embodiment, the processor 320 may set tₖ based on an input of setting tₖ which is received through an input interface (not illustrated). For example, tₖ may be set to a predetermined time based on an input of setting tₖ to a predetermined time (for example, 5 ms), which is received through the input interface. In addition, tₖ may be set to increase as k increases based on an input of setting tₖ to satisfy tₖ > tₖ₋₁, which is received through the input interface.

According to an embodiment, the processor 320 may automatically set tₖ by taking into account the time at which the contrast agent rises in the region of interest of the object after the contrast agent is destroyed, the property of the object, and the number of cumulative ultrasound contrast agent images necessary for lesion diagnosis.

The processor 320 may set the number fₖ of contrast agent signals detected by the probe 310 after the contrast agent is destroyed. According to an embodiment, the processor 320 may set fₖ based on an input of setting fₖ, which is received through the input interface. For example, fₖ may be set to a predetermined number based on an input of setting fₖ to a predetermined number (for example, 5), which is received through the input interface. In addition, fₖ may be set to increase as k increases based on an input of setting fₖ to satisfy fₖ > fₖ₋₁, which is received through the input interface.

According to an embodiment, the processor 320 may automatically set fₖ by taking into account the time at which the contrast agent rises to the region of interest of the object after the contrast agent is destroyed, the property of the object, and the number of cumulative ultrasound contrast agent images necessary for lesion diagnosis.

According to an embodiment, when the processor 320 differently sets tₖ and fₖ, the probe 310 may detect contrast agent signals having different degrees of the rising of the contrast agent in the region of interest of the object. When the processor 320 sets tₖ to be longer or sets fₖ to be greater, the time for the probe 310 to detect the contrast agent signal after the contrast agent is destroyed is prolonged. In addition, as the time passes by, the contrast agent moves through the bloodstream and the degree of the rising of the contrast agent is changed. Therefore, the processor 320 sets tₖ to be gradually longer or sets fₖ to be gradually greater, so that the probe 310 detects a plurality of contrast agent signals after the contrast agent is destroyed. Thus, the probe 310 may detect contrast agent signals having different degrees of the rising of the contrast agent in the region of interest of the object as the time passes by.

The processor 320 may generate the ultrasound contrast agent image by using the contrast agent signal detected by the probe 310. According to an embodiment, the processor 320 may generate a plurality of ultrasound contrast agent images by using a plurality of contrast agent signals detected during the time interval of tₖ by the probe 310 after the contrast agent is destroyed. Since the probe 310 is capable of, under the control of the processor 320, repeating the process of detecting the contrast agent signals n times from k=1 to k=n during the time interval of tₖ after the contrast agent is destroyed, the processor 320 may generate the ultrasound contrast agent images by using the contrast agent signals detected through the probe 310 with respect to each of n-time repetitions.

According to an embodiment, the processor 320 may generate fₖ ultrasound contrast agent images by using fₖ contrast agent signals detected by the probe 310 after the contrast agent is destroyed. Since the probe 310 is capable of, under the control of the processor 320, repeating the process of detecting fₖ contrast agent signals n times from k=1 to k=n after the contrast agent is destroyed, the processor 320 may generate fₖ ultrasound contrast agent images by using the contrast agent signals detected by the probe 310 with respect to each of n-time repetitions.

The processor 320 may generate a cumulative ultrasound contrast agent image by accumulating a plurality of ultrasound contrast agent images. According to an embodiment, the processor 320 may generate a cumulative ultrasound contrast agent image by accumulating a plurality of ultrasound contrast agent images generated by using a plurality of contrast agent signals detected during the time interval of tₖ by the probe 310 after the contrast agent is destroyed. In addition, the processor 320 may generate n cumulative ultrasound contrast agent images by performing, n times from k=1 to k=n, a process of generating a cumulative ultrasound contrast agent image by accumulating a plurality of ultrasound contrast agent images generated by using a plurality of contrast agent signals detected during the time interval of tₖ by the probe 310 after the contrast agent is destroyed.

According to an embodiment, the processor 320 may generate a cumulative ultrasound contrast agent image by accumulating fₖ ultrasound contrast agent images generated by using fₖ contrast agent signals detected by the probe 310 after the contrast agent is destroyed. In addition, the processor 320 may generate n cumulative ultrasound contrast agent images by performing, n times from k=1 to k=n, a process of generating a cumulative ultrasound contrast agent image by accumulating fₖ ultrasound contrast agent images generated by using fₖ contrast agent signals detected by the probe 310 after the contrast agent is destroyed.

The n cumulative ultrasound contrast agent images generated herein may be ultrasound contrast agent images having different degrees of the rising of the contrast agent in the region of interest of the object according to tₖ or fₖ set by the processor 320. In particular, when the processor 320 sets tₖ to be gradually longer or sets fₖ to be gradually greater so that the probe 310 detects a plurality of contrast agent signals after the contrast agent is destroyed, the processor 320 may use the detected contrast agent signals to generate, step by step, cumulative ultrasound contrast agent images having different degrees of the rising of the contrast agent in the region of interest of the object as the times passes by.

That is, according to an embodiment, the position at which the contrast agent begins to rise in the region of interest of the object and the shape thereof may be identified through the cumulative ultrasound contrast agent images having different degrees of the rising of the contrast agent in the region of interest of the object as the time passes by. Information necessary for lesion diagnosis may be obtained by identifying the position at which the contrast agent begins to rise and the shape thereof. For example, in the case of focal nodular hyperplasia (FNH) and adenoma, which are the lesions present in the liver, the two lesions are difficult to distinguish from each other since the two lesions show very similar characteristics. However, for the contrast agent images, the two lesions show distinctive characteristics in terms of the position at which the contrast agent rises and the shape thereof. Therefore, the two lesions may be distinguished and diagnosed by identifying the distinctive characteristics. Therefore, according to the disclosed embodiment, the lesions may be easily diagnosed by identifying the position at which the contrast agent begins to rise and the shape thereof.

According to an embodiment, the processor 320 may generate a cumulative ultrasound contrast agent image by accumulating all or part of the generated n cumulative ultrasound contrast agent images again (indicating first to n^{th} cumulative ultrasound contrast agent images in the disclosure). For example, the processor 320 may additionally generate n cumulative ultrasound contrast agent images (indicating first to n^{th} total cumulative ultrasound contrast agent images in the disclosure) by performing, n times from k=1 to k=n, a process of generating a cumulative ultrasound contrast agent image by accumulating first to k^{th} cumulative ultrasound contrast agent images.

When the first to k^{th} cumulative ultrasound contrast agent images are cumulative ultrasound contrast agent images having stepwise different degrees of the rising of the contrast agent in the region of interest of the object as the time passes by, the additionally generated first to n^{th} total cumulative ultrasound contrast agent images may also be cumulative ultrasound contrast agent images having stepwise different degrees of the rising of the contrast agent in the region of interest of the object as the time passes by.

According to an embodiment, the additionally generated n cumulative ultrasound contrast agent images (the first to n^{th} total cumulative ultrasound contrast agent images) may be images obtained by accumulating contrast agent images with respect to a short time at which the contrast agent begins to rise immediately after the contrast agent is destroyed. Therefore, the additionally generated n cumulative ultrasound contrast agent images are images in which the position where the contrast agent begins to initially rise are shown more clearly than other positions within the region of interest of the object, and the position where the contrast agent begins to rise may be more clearly identified through these images.

According to an embodiment, the processor 320 may generate a cumulative ultrasound contrast agent image by selectively accumulating only part of a plurality of generated ultrasound contrast agent images. For example, the processor 320 may generate a cumulative ultrasound contrast agent image by selecting part of a plurality of ultrasound contrast agent images (for example, images having little shaking according to the movement of the object) generated by using a plurality of ultrasound contrast agent signals detected by the probe 310.

The processor 320 may set the number n of times of repetitions of a series of processes. According to an embodiment, the processor 320 may set n based on an input of setting n, which is received through the input interface. In addition, according to an embodiment, the processor 320 may automatically set n by taking into account the time at which the contrast agent rises in the region of interest of the object after the contrast agent is destroyed, the property of the object, and the number of cumulative ultrasound contrast agent images necessary for lesion diagnosis.

The processor 320 may correct the shaking of a plurality of ultrasound contrast agent images according to the movement of the object. The probe 310 may detect a plurality of contrast agent signals during the time interval of tₖ under the control of the processor 320, or the object may move during the detection of fₖ contrast agent signals. Therefore, the plurality of ultrasound contrast agent images generated by the processor 320 by using the plurality of contrast agent signals detected by the probe 310 may be shaken by the influence of the movement of the object. In this case, the processor 320 may set the image serving as the reference among the plurality of generated ultrasound contrast agent images and correct the shaking of the other ultrasound contrast agent images.

According to an embodiment, before accumulating the ultrasound contrast agent images, the processor 320 may correct the shaking of the ultrasound contrast agent images according to the movement of the object, so as to generate much clearer cumulative ultrasound contrast agent images.

The processor 320 may generate one video edited so that a plurality of cumulative ultrasound contrast agent images are sequentially reproduced. According to an embodiment, the processor 320 may generate one video edited so that cumulative ultrasound contrast agent images (including total cumulative ultrasound contrast agent images in the disclosure) having different degrees of the rising of the contrast agent in the region of interest of the object are sequentially reproduced as the time passes by. The position at which the contrast agent begins to rise in the region of interest of the object and the shape of the rising of the contrast agent as the time passes by may be identified through the generated video.

The processor 320 may store the ultrasound contrast agent image in a storage (not illustrated). According to an embodiment, the processor 320 may store, in the storage, the ultrasound contrast agent images generated by using the signals detected by the probe 310, the cumulative ultrasound contrast agent images, the total cumulative ultrasound contrast agent images, and the video obtained by editing the cumulative ultrasound contrast agent images.

The display 330 may display the ultrasound contrast agent image. According to an embodiment, the display 330 may sequentially display the n cumulative ultrasound contrast agent images generated by the processor 320 on one screen at the same time. For example, under the control of the processor 320, the display 330 may sequentially display the cumulative ultrasound contrast agent images having different degrees of the rising of the contrast agent in the region of interest of the object in the order of time lapse as the time passes by.

According to an embodiment, the display 330 may display one video edited so that the cumulative ultrasound contrast agent images are sequentially reproduced by the processor 320.

FIG. 4 is a flowchart of a method by which the ultrasound imaging apparatus 300 generates and displays a cumulative ultrasound contrast agent image of a region of interest of an object, according to an embodiment.

Referring to FIG. 4, a method of generating and displaying a cumulative ultrasound contrast agent image of a region of interest of an object, according to an embodiment, may include operations processed in time series by the ultrasound imaging apparatus 300 illustrated in FIG. 3. Therefore, even though omitted below, the description provided for the ultrasound imaging apparatus 300 illustrated in FIG. 3 may be applied to the method of generating and displaying the cumulative ultrasound contrast agent image of the region interest of the object as illustrated in FIG. 4.

In operation 410, the ultrasound imaging apparatus 300 transmits an ultrasound signal for destroying a contrast agent to a region of interest of an object into which the contrast agent is injected. A sound pressure of the transmitted ultrasound signal may have an energy level capable of destroying the contrast agent. The contrast agent injected into the region of interest of the object may be destroyed by the transmitted ultrasound signal.

In operation 420, the ultrasound imaging apparatus 300 acquires a plurality of ultrasound contrast agent images of the region of interest of the object during the time interval of tₖ. At this time, the ultrasound imaging apparatus 300 may acquire a plurality of ultrasound contrast agent images based on a plurality of contrast agent signals for the region of interest of the object detected during the time interval of tₖ.

In operation 430, the ultrasound imaging apparatus 300 generates a k^{th} cumulative ultrasound contrast agent image by accumulating the acquired ultrasound contrast agent images.

In operation 440, the ultrasound imaging apparatus 300 determines whether operations 410 to 430 are repeated n times (that is, whether k=n). In operation 450, when the number of times of repetitions is not n, the ultrasound imaging apparatus 300 sets tₖ₊₁ to satisfy tₖ₊₁ > tₖ and repeats operations 410 to 430.

In operation 460, when the ultrasound imaging apparatus 300 repeats operations 410 to 430 n times, the ultrasound imaging apparatus 300 displays the n cumulative ultrasound contrast agent images.

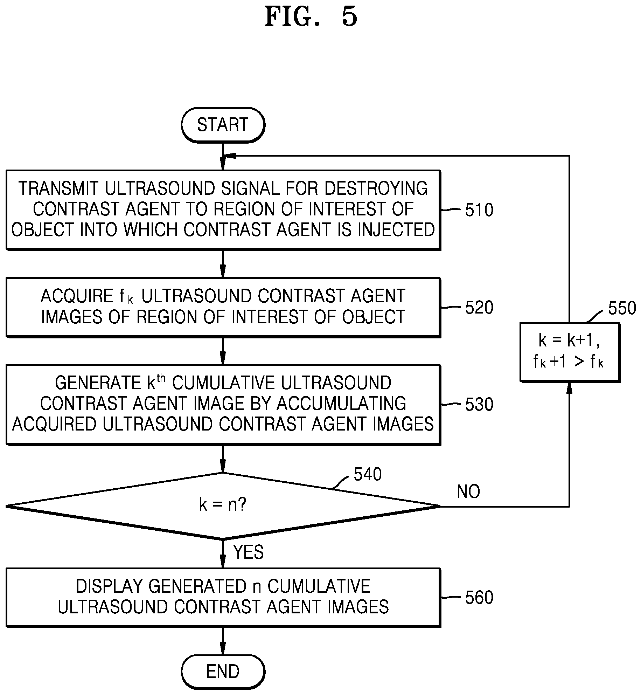

FIG. 5 is a flowchart of a method by which the ultrasound imaging apparatus 300 generates and displays a cumulative ultrasound contrast agent image of a region of interest of an object, according to an embodiment.

Referring to FIG. 5, a method of generating and displaying a cumulative ultrasound contrast agent image of a region of interest of an object, according to another embodiment, may include operations processed in time series by the ultrasound imaging apparatus 300 illustrated in FIG. 3. Therefore, even though omitted below, the description provided for the ultrasound imaging apparatus 300 illustrated in FIG. 3 may be applied to the method of generating and displaying the cumulative ultrasound contrast agent image of the region interest of the object as illustrated in FIG. 5.

In operation 510, the ultrasound imaging apparatus 300 transmits an ultrasound signal for destroying a contrast agent to a region of interest of an object into which the contrast agent is injected. A sound pressure of the transmitted ultrasound signal may have an energy level capable of destroying the contrast agent. The contrast agent injected into the region of interest of the object may be destroyed by the transmitted ultrasound signal.

In operation 520, the ultrasound imaging apparatus 300 acquires fₖ ultrasound contrast agent images of the region of interest of the object. At this time, the ultrasound imaging apparatus 300 may acquire fₖ ultrasound contrast agent images based on fₖ contrast agent signals detected for the region of interest of the object.

In operation 530, the ultrasound imaging apparatus 300 generates a k^{th} cumulative ultrasound contrast agent image by accumulating the acquired fₖ ultrasound contrast agent images.

In operation 540, the ultrasound imaging apparatus 300 determines whether operations 510 to 530 are repeated n times (that is, whether k=n). In operation 550, when the number of times of repetitions is not n, the ultrasound imaging apparatus 300 sets fₖ₊₁ to satisfy fₖ₊₁ > fₖ and repeats operations 510 to 530.

In operation 560, when the ultrasound imaging apparatus 300 repeats operations 510 to 530 n times, the ultrasound imaging apparatus 300 displays n cumulative ultrasound contrast agent images.

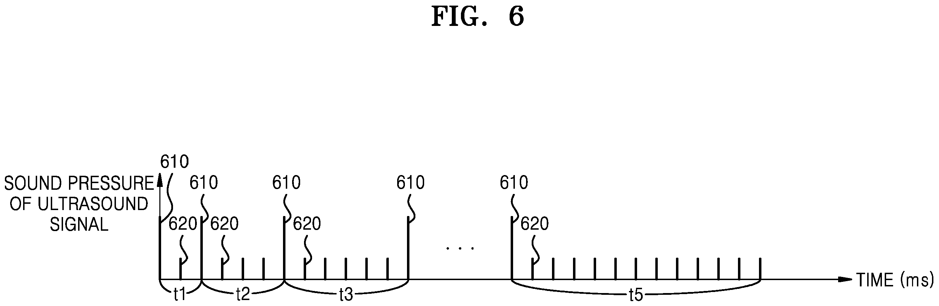

FIG. 6 is a diagram illustrating an example of an ultrasound signal transmitted to an object by the ultrasound imaging apparatus 300, according to an embodiment.

As illustrated in FIG. 6, the ultrasound imaging apparatus 300 according to the embodiment transmits, to an object, an ultrasound signal 610 for destroying a contrast agent and an ultrasound signal 620 for detecting a contrast agent signal (that is, an echo signal). The ultrasound signal 610 for destroying the contrast agent has an energy level capable of destroying the contrast agent and is illustrated in FIG. 6 as having a high sound pressure. The ultrasound signal 620 for detecting the contrast agent signal has an energy level capable of detecting the contrast agent signal and is illustrated in FIG. 6 as having a relative low sound pressure, as compared with the ultrasound signal 610 for destroying the contrast agent.

In addition, as illustrated in FIG. 6, the ultrasound imaging apparatus 300 according to the embodiment transmits the ultrasound signal 610 for destroying the contrast agent to the region of interest of the object at time intervals of tₖ, and transmits the ultrasound signal 620 for detecting the contrast agent signal to the region of interest of the object during the time interval of tₖ after the ultrasound signal 620 for destroying the contrast agent is transmitted (that is, after the contrast agent is destroyed). The ultrasound imaging apparatus 300 may detect the contrast agent signal as the echo signal for each transmitted ultrasound signal 620 for detecting the contrast agent signal.

FIG. 6 illustrates an example in which the ultrasound imaging apparatus 300 sets tₖ to satisfy tₖ₊₁ > tₖ, according to an embodiment. It can be seen from FIG. 6 that as k increases, the time interval tₖ also increases, and as the time interval tₖ increases, more ultrasound signals 620 for detecting the contrast agent signals are transmitted. Therefore, the example illustrated in FIG. 6 may be an example of an ultrasound signal transmitted to an object by the ultrasound imaging apparatus 300 when the ultrasound imaging apparatus 300 generates, step by step, cumulative ultrasound contrast agent images having different degrees of the rising of the contrast agent in the region of interest of the object as the time passes by.

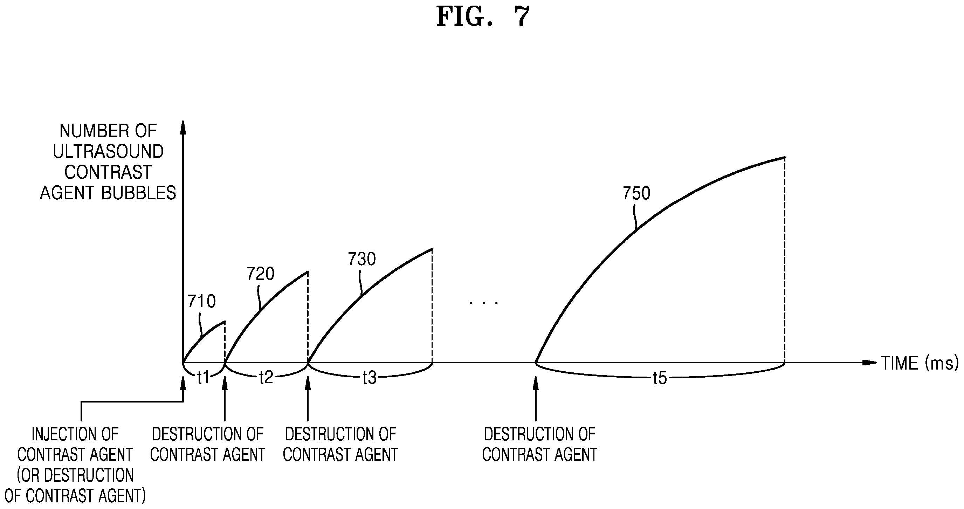

FIG. 7 is a diagram illustrating an example in which the number of ultrasound contrast agent bubbles of an object is changed as the ultrasound imaging apparatus 300 transmits an ultrasound signal to an object.

As illustrated in FIG. 7, the ultrasound imaging apparatus 300 according to the embodiment transmits an ultrasound signal for destroying a contrast agent to a region of interest of an object at time intervals of tₖ, so as to destroy the contrast agent of the region of interest of the object at the time intervals of tₖ.

Referring to FIG. 7, the number of ultrasound contrast agent bubbles in the region of interest of the region approaches to zero when the ultrasound signal for destroying the contrast agent is transmitted to the region of interest of the object and the contrast agent is destroyed, and the number of ultrasound contrast agent bubbles in the region of interest of the object is increased since the ultrasound contrast agent rises again during the time interval of tₖ after the contrast agent is destroyed.

FIG. 7 illustrates an example in which the ultrasound imaging apparatus 300 sets tₖ to satisfy tₖ₊₁ > tₖ, according to an embodiment. It can be seen from FIG. 7 that as k increases, the time interval tₖ also increases, and as the time interval tₖ increases, a larger number of ultrasound contrast agent bubbles rise. Therefore, the example illustrated in FIG. 7 may be an example in which the number of ultrasound contrast agent bubbles is changed when the ultrasound imaging apparatus 300 generates, step by step, cumulative ultrasound contrast agent images having different degrees of the rising of the contrast agent in the region of interest of the object as the time passes by.

However, the example illustrated in FIG. 7 is merely an example showing that the degree of the rising of the contrast agent in the region of interest of the object is changed according to tₖ, and the aspect of the rising of the contrast agent after the contrast agent is destroyed may be differently shown by the region of interest of the object, the type of the contrast agent, and the like.

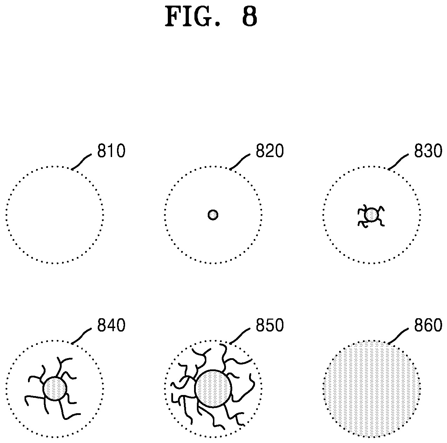

FIG. 8 is a diagram illustrating an example in which the ultrasound imaging apparatus 300 displays a cumulative ultrasound contrast agent image of a region of interest of an object, according to an embodiment.

Referring to FIG. 8, the ultrasound imaging apparatus 300 according to the embodiment displays an ultrasound image 810 before a contrast agent is injected as a control group, and sequentially displays cumulative ultrasound contrast agent images 820 to 860 having different degrees of the rising of the contrast agent in the region of interest of the object in the order of time lapse as the time passes by. The position at which the contrast agent begins to rise in the region of interest of the object and the shape of the rising of the contrast agent as the time passes by may be identified through the cumulative ultrasound contrast agent images 820 to 860 illustrated in FIG. 8.

According to an embodiment, since the cumulative ultrasound contrast agent images having different degrees of the rising of the contrast agent in the region of interest of the object as the time passes by are sequentially displayed in the order of time lapse, the position at which the contrast agent begins to initially rise in the region of interest of the object may be clearly identified, and the shape of the rising of the contrast agent may also be clearly identified.

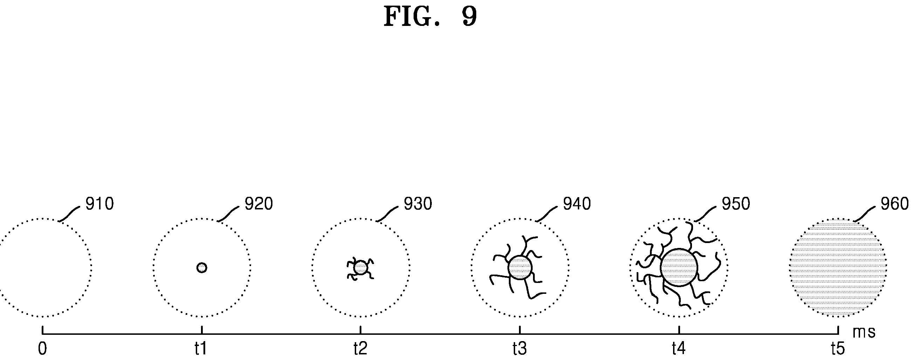

FIG. 9 is a diagram illustrating an example of a video edited so that the ultrasound imaging apparatus 300 sequentially reproduces a plurality of cumulative ultrasound contrast agent images of a region of interest of an object, according to an embodiment.

When a video is started, the ultrasound imaging apparatus 300 according to the embodiment may reproduce an ultrasound image 910 before a contrast agent is injected as a control group. The ultrasound imaging apparatus 300 may edit the video so that cumulative ultrasound contrast agent images 920 to 960 having different degrees of the rising of the contrast agent in the region of interest of the object as the time passes by are sequentially reproduced (for example, reproduced at the corresponding elapsed time (for example, tₖ)). An example of the edited video is illustrated in FIG. 9. The position at which the contrast agent begins to rise in the region of interest of the object and the shape of the rising of the contrast agent as the time passes by may be identified through the video obtained by editing the cumulative ultrasound contrast agent images 920 to 960 illustrated in FIG. 9.

According to an embodiment, the position at which the contrast agent begins to initially rise in the region of interest of the object and the shape of the rising of the contrast agent may be clearly identified through the video edited so that the cumulative ultrasound contrast agent images having different degrees of the rising of the contrast agent in the region of interest of the object as the time passes by are sequentially displayed in the order of time lapse.

The disclosed embodiments may be embodied in the form of a non-transitory computer-readable recording medium having recorded thereon instructions and data executable by a computer. The instructions may be stored in the form of a program code, and when executed by a processor, a predetermined program module may be generated to perform a predetermined operation. In addition, the instructions, when executed by the processor, may perform certain operations of the disclosed embodiments.

It should be understood that embodiments described herein should be considered in a descriptive sense only and not for purposes of limitation. Descriptions of features or aspects within each embodiment should typically be considered as available for other similar features or aspects in other embodiments.

## Claims

1. An ultrasound imaging apparatus (100, 100a, 100b, 100c, 300) comprising:
a probe (20, 310) configured to transmit an ultrasound signal (610, 620) to an object (10) and detect an echo signal;
one or more processors (120, 130, 320) configured to transmit an ultrasound signal (610, 620) for destroying a contrast agent through the probe (20, 310) to a region of interest of the object (10) into which the contrast agent is injected, acquire a plurality of ultrasound contrast agent images of the region of interest of the object (10) at time intervals of tₖ (k⊆1, 2, ..., n, and n is an integer of 2 or more), and generate a k^{th} cumulative ultrasound contrast agent image by accumulating the plurality of acquired ultrasound contrast agent images at every time intervals of tₖ to generate n cumulative ultrasound contrast agent images (820 - 860, 920 - 960); and
a display (121, 122, 140, 330) configured to display the generated n cumulative ultrasound contrast agent images (820 - 860, 920 - 960) sequentially in the order of time lapse,
wherein tₖ satisfies tₖ > tₖ₋₁.

2. The ultrasound imaging apparatus (100, 100a, 100b, 100c, 300) of claim 1, wherein a sound pressure of the ultrasound signal (610, 620) is determined based on an energy level for destroying the contrast agent.

3. The ultrasound imaging apparatus (100, 100a, 100b, 100c, 300) of claim 1, further comprising an input interface (170) configured to receive a user input of setting tₖ and n,
wherein the one or more processors (120, 130, 320) are further configured to set tₖ and n based on the user input.

4. The ultrasound imaging apparatus (100, 100a, 100b, 100c, 300) of claim 1, further comprising a storage (150) storing the plurality of acquired ultrasound contrast agent images and the k^{th} cumulative ultrasound contrast agent image.

5. The ultrasound imaging apparatus (100, 100a, 100b, 100c, 300) of claim 1, wherein the one or more processors (120, 130, 320) are further configured to correct shaking of the plurality of ultrasound contrast agent images according to movement of the object (10).

6. The ultrasound imaging apparatus (100, 100a, 100b, 100c, 300) of claim 1, wherein the one or more processors (120, 130, 320) are further configured to generate one video edited so that the n cumulative ultrasound contrast agent images (820 - 860, 920 - 960) are sequentially reproduced, and
the display (121, 122, 140, 330) is further configured to display the video.

7. The ultrasound imaging apparatus (100, 100a, 100b, 100c, 300) of claim 1, wherein the one or more processors (120, 130, 320) are further configured to generate first to n^{th} total cumulative ultrasound contrast agent images (820 - 860, 920 - 960) by performing, n times from k=1 to k=n, a process of generating a k^{th} total cumulative ultrasound contrast agent image obtained by accumulating first to k^{th} cumulative ultrasound contrast agent images (820 - 860, 920 - 960), and
the display (121, 122, 140, 330) is further configured to display the first to n^{th} total cumulative ultrasound contrast agent images (820 - 860, 920 - 960).

8. The ultrasound imaging apparatus (100, 100a, 100b, 100c, 300) of claim 7, wherein
the one or more processors (120, 130, 320) are further configured to generate one video edited so that the first to n^{th} total cumulative ultrasound contrast agent images (820 - 860, 920 - 960) are sequentially reproduced, and
the display (121, 122, 140, 330) is further configured to display the video.

9. An ultrasound imaging apparatus (100, 100a, 100b, 100c, 300) comprising:
a probe (20, 310) configured to transmit an ultrasound signal (610, 620) to an object (10) and detect an echo signal;
one or more processors (120, 130, 320) configured to transmit an ultrasound signal (610, 620) for destroying a contrast agent through the probe (20, 310) to a region of interest of the object (10) into which the contrast agent is injected, acquire fₖ ultrasound contrast agent images of the region of interest of the object (10) (k⊆1, 2, ..., n, and n is an integer of 2 or more), and generate n cumulative ultrasound contrast agent images by performing, n times from k=1 to k=n, a process of generating a k^{th} cumulative ultrasound contrast agent image by accumulating the acquired fₖ ultrasound contrast agent images; and
a display (121, 122, 140, 330) configured to display the generated n cumulative ultrasound contrast agent images (820 - 860, 920 - 960) sequentially in the order of time lapse,
wherein fₖ satisfies fₖ > fₖ₋₁.

10. The ultrasound imaging apparatus (100, 100a, 100b, 100c, 300) of claim 9, wherein one or more processors (120, 130, 320) are further configured to generate one video edited so that the n cumulative ultrasound contrast agent images are sequentially reproduced, and
the display (121, 122, 140, 330) is further configured to display the video.

11. The ultrasound imaging apparatus (100, 100a, 100b, 100c, 300) of claim 9, wherein the one or more processors (120, 130, 320) are further configured to generate first to n^{th} total cumulative ultrasound contrast agent images by performing, n times from k=1 to k=n, a process of generating a k^{th} total cumulative ultrasound contrast agent image obtained by accumulating first to k^{th} cumulative ultrasound contrast agent images, and
the display (121, 122, 140, 330) is further configured to display the first to n^{th} total cumulative ultrasound contrast agent images (820 - 860, 920 - 960).

12. A method of controlling an ultrasound imaging apparatus (100, 100a, 100b, 100c, 300), the method comprising:
transmitting an ultrasound signal (610, 620) for destroying a contrast agent through a probe (20, 310) to a region of interest of the object (10) into which the contrast agent is injected and acquiring a plurality of ultrasound contrast agent images of the region of interest of the object (10) at time intervals of tₖ (k⊆1, 2, ..., n, and n is an integer of 2 or more), generating a k^{th} cumulative ultrasound contrast agent image by accumulating the plurality of acquired ultrasound contrast agent images at every time intervals of tₖ to generate n cumulative ultrasound contrast agent images (820 - 860, 920 - 960); and
displaying the generated n cumulative ultrasound contrast agent images (820-860,920-960), sequentially in the order of time lapse;
wherein tₖ satisfies tₖ > tₖ₋₁.

13. The method of claim 12, further comprising:
generating one video edited so that the n cumulative ultrasound contrast agent images are sequentially reproduced; and
displaying the video.

14. The method of claim 12, further comprising:
generating first to n^{th} total cumulative ultrasound contrast agent images by performing, n times from k=1 to k=n, a process of generating a k^{th} total cumulative ultrasound contrast agent image obtained by accumulating first to k^{th} cumulative ultrasound contrast agent images; and
displaying the first to n^{th} total cumulative ultrasound contrast agent images.

15. A computer program product comprising a computer-readable storage medium storing instructions for performing the method of claim 12.

## Patentansprüche

1. Ultraschallbildgebungsvorrichtung (100, 100a, 100b, 100c, 300), die Folgendes umfasst:
eine Sonde (20, 310), die dazu konfiguriert ist, ein Ultraschallsignal (610, 620) an ein Objekt (10) zu übertragen und ein Echosignal zu erfassen;
wenigstens einen Prozessor (120, 130, 320), der zu Folgendem konfiguriert ist: Übertragen eines Ultraschallsignals (610, 620) zum Zerstören eines Kontrastmittels durch die Sonde (20, 310) an eine Region von Interesse des Objekts (10), in die das Kontrastmittel injiziert worden ist, Erhalten einer Vielzahl von Ultraschallkontrastmittelbildern der Region von Interesse des Objekts (10) in Zeitintervallen von tₖ (wobei k⊆1, 2, ..., n, und wobei es sich bei n um eine ganze Zahl von 2 oder mehr handelt), und Erzeugen eines k-ten kumulativen Ultraschallkontrastmittelbilds durch Akkumulieren der Vielzahl von erhaltenen Ultraschallkontrastmittelbildern an jedem der Zeitintervalle von tₖ, um n kumulative Ultraschallkontrastmittelbilder (820 - 860, 920 - 960) zu erzeugen; und
eine Anzeige (121, 122, 140, 440), die dazu konfiguriert ist, die erzeugten n kumulativen Ultraschallkontrastmittelbilder (820 - 860, 920 - 960) nacheinander in chronologischer Reihenfolge anzuzeigen,
wobei tₖFolgendes erfüllt: tₖ > tₖ₋₁.

2. Ultraschallbildgebungsvorrichtung (100, 100a, 100b, 100c, 300) nach Anspruch 1, wobei ein Schalldruck des Ultraschallsignals (610, 620) basierend auf einem Energieniveau zum Zerstören des Kontrastmittels bestimmt wird.

3. Ultraschallbildgebungsvorrichtung (100, 100a, 100b, 100c, 300) nach Anspruch 1, die weiterhin eine Eingabeschnittstelle (170) umfasst, die dazu konfiguriert ist, eine Benutzereingabe zum Einstellen von tₖ und n zu empfangen,
wobei der wenigstens eine Prozessor (120, 130, 320) weiterhin dazu konfiguriert ist, tₖ und n basierend auf der Benutzereingabe einzustellen.

4. Ultraschallbildgebungsvorrichtung (100, 100a, 100b, 100c, 300) nach Anspruch 1, die weiterhin einen Speicher (150) umfasst, in dem die Vielzahl von erhaltenen Ultraschallkontrastmittelbildern und das k-te kumulative Ultraschallkontrastmittelbild gespeichert sind.

5. Ultraschallbildgebungsvorrichtung (100, 100a, 100b, 100c, 300) nach Anspruch 1, wobei der wenigstens eine Prozessor (120, 130, 320) weiterhin dazu konfiguriert ist, ein Wackeln der Vielzahl von Ultraschallkontrastmittelbildern wegen der Bewegung des Objekts (10) zu korrigieren.

6. Ultraschallbildgebungsvorrichtung (100, 100a, 100b, 100c, 300) nach Anspruch 1, wobei der wenigstens eine Prozessor (120, 130, 320) weiterhin dazu konfiguriert ist, ein Video zu erzeugen, das so bearbeitet ist, dass die n kumulativen Ultraschallkontrastmittelbilder (820 - 860, 920 - 960) nacheinander wiedergegeben werden, und
wobei die Anzeige (121, 122, 140, 330) weiterhin dazu konfiguriert ist, das Video anzuzeigen.

7. Ultraschallbildgebungsvorrichtung (100, 100a, 100b, 100c, 300) nach Anspruch 1, wobei der wenigstens eine Prozessor (120, 130, 320) weiterhin dazu konfiguriert ist, erste bis n-te totale kumulative Ultraschallkontrastmittelbilder (820 - 860, 920 - 960) zu erzeugen mittels Durchführen, und zwar n Mal von k=1 bis k=n, eines Prozesses zur Erzeugung eines k-ten totalen kumulativen Ultraschallkontrastmittelbildes, das erhalten wird durch Akkumulieren erster bis k-ter kumulativer Ultraschallkontrastmittelbilder (820 - 860, 920 - 960), und wobei die Anzeige (121, 122, 140, 330) weiterhin dazu konfiguriert ist, die ersten bis n-ten totalen kumulativen Ultraschallkontrastmittelbilder (820 - 860, 920 - 960) anzuzeigen.

8. Ultraschallbildgebungsvorrichtung (100, 100a, 100b, 100c, 300) nach Anspruch 7, wobei der wenigstens eine Prozessor (120, 130, 320) weiterhin dazu konfiguriert ist, ein Video zu erzeugen, das so bearbeitet ist, dass die ersten bis n-ten totalen kumulativen Ultraschallkontrastmittelbilder (820 - 860, 920 - 960) nacheinander wiedergegeben werden, und
wobei die Anzeige (121, 122, 140, 330) weiterhin dazu konfiguriert ist, das Video anzuzeigen.

9. Ultraschallbildgebungsvorrichtung (100, 100a, 100b, 100c, 300), die Folgendes umfasst:
eine Sonde (20, 310), die dazu konfiguriert ist, ein Ultraschallsignal (610, 620) an ein Objekt (10) zu übertragen und ein Echosignal zu erfassen;
wenigstens einen Prozessor (120, 130, 320), der zu Folgendem konfiguriert ist: Übertragen eines Ultraschallsignals (610, 620) zum Zerstören eines Kontrastmittels durch die Sonde (20, 310) an eine Region von Interesse des Objekts (10), in die das Kontrastmittel injiziert worden ist, Erhalten von fₖ Ultraschallkontrastmittelbildern der Region von Interesse des Objekts (10) (wobei k⊆1, 2, ..., n, und wobei es sich bei n um eine ganze Zahl von 2 oder mehr handelt), und Erzeugen von n kumulativen Ultraschallkontrastmittelbildern mittels Durchführen, und zwar n Mal von k=1 bis k=n, eines Prozesses zur Erzeugung eines k-ten kumulativen Ultraschallkontrastmittelbildes durch Akkumulieren der erhaltenen fₖ Ultraschallkontrastmittelbilder; und
eine Anzeige (121, 122, 140, 330), die dazu konfiguriert ist, die erzeugten n kumulativen Ultraschallkontrastmittelbilder (820 - 860, 920 - 960) nacheinander in chronologischer Reihenfolge anzuzeigen,
wobei fₖ Folgendes erfüllt: fₖ > fₖ₋₁.

10. Ultraschallbildgebungsvorrichtung (100, 100a, 100b, 100c, 300) nach Anspruch 9, wobei wenigstens ein Prozessor (120, 130, 320) weiterhin dazu konfiguriert ist, ein Video zu erzeugen, das so bearbeitet ist, dass die n kumulativen Ultraschallkontrastmittelbilder nacheinander wiedergegeben werden, und
wobei die Anzeige (121, 122, 140, 330) weiterhin dazu konfiguriert ist, das Video anzuzeigen.

11. Ultraschallbildgebungsvorrichtung (100, 100a, 100b, 100c, 300) nach Anspruch 9, wobei der wenigstens eine Prozessor (120, 130, 320) weiterhin dazu konfiguriert ist, erste bis n-te totale kumulative Ultraschallkontrastmittelbilder zu erzeugen mittels Durchführen, und zwar n Mal von k=1 bis k=n, eines Prozesses zur Erzeugung eines k-ten totalen kumulativen Ultraschallkontrastmittelbildes, das erhalten wird durch Akkumulieren erster bis k-ter kumulativer Ultraschallkontrastmittelbilder, und
wobei die Anzeige (121, 122, 140, 330) weiterhin dazu konfiguriert ist, die ersten bis n-ten totalen kumulativen Ultraschallkontrastmittelbilder (820 - 860, 920 - 960) anzuzeigen.

12. Verfahren zur Steuerung einer Ultraschallbildgebungsvorrichtung (100, 100a, 100b, 100c, 300), wobei das Verfahren Folgendes umfasst:
Übertragen eines Ultraschallsignals (610, 620) zum Zerstören eines Kontrastmittels durch eine Sonde (20, 310) an eine Region von Interesse des Objekts (10), in die das Kontrastmittel injiziert worden ist, und Erhalten einer Vielzahl von Ultraschallkontrastmittelbildern der Region von Interesse des Objekts (10) in Zeitintervallen von tₖ (wobei kcl, 2, ..., n, und wobei es sich bei n um eine ganze Zahl von 2 oder mehr handelt), Erzeugen eines k-ten kumulativen Ultraschallkontrastmittelbilds durch Akkumulieren der Vielzahl von erhaltenen Ultraschallkontrastmittelbildern an jedem der Zeitintervalle von tₖ, um n kumulative Ultraschallkontrastmittelbilder (820 - 860, 920 - 960) zu erzeugen; und
Anzeigen der erzeugten n kumulativen Ultraschallkontrastmittelbilder (820 - 860, 920 - 960) nacheinander in chronologischer Reihenfolge;
wobei tₖ Folgendes erfüllt: tₖ > tₖ₋₁.

13. Verfahren nach Anspruch 12, das weiterhin Folgendes umfasst:
Erzeugen eines Videos, das so bearbeitet ist, dass die n kumulativen Ultraschallkontrastmittelbilder nacheinander wiedergegeben werden; und
Anzeigen des Videos.

14. Verfahren nach Anspruch 12, das weiterhin Folgendes umfasst:
Erzeugen von ersten bis n-ten totalen kumulativen Ultraschallkontrastmittelbildern mittels Durchführen, und zwar n Mal von k=1 bis k=n, eines Prozesses zur Erzeugung eines k-ten totalen kumulativen Ultraschallkontrastmittelbildes, das erhalten wird durch Akkumulieren von ersten bis k-ten kumulativen Ultraschallkontrastmittelbildern; und
Anzeigen der ersten bis k-ten totalen kumulativen Ultraschallkontrastmittelbilder.

15. Computerprogrammprodukt, das ein computerlesbares Speichermedium umfasst, in dem Anweisungen zur Durchführung des Verfahrens nach Anspruch 12 gespeichert sind.

## Revendications

1. Dispositif d'imagerie échographique (100, 100a, 100b, 100c, 300) comprenant :
une sonde (20, 310) conçue pour émettre un signal ultrasonore (610, 620) vers un objet (10) et pour détecter un signal d'écho ;
un ou plusieurs processeurs (120, 130, 320) conçus pour émettre un signal ultrasonore (610, 620), visant à détruire un agent de contraste, par la sonde (20, 310) vers une région d'intérêt de l'objet (10) dans laquelle l'agent de contraste a été injecté, pour acquérir une pluralité d'images d'échographie de contraste de la région d'intérêt de l'objet (10) à des intervalles de temps tₖ (k⊆1, 2, ..., n, et n est un entier valant deux ou plus), et pour générer une k^{ième} image d'échographie de contraste cumulative par cumulation de la pluralité d'images d'échographie de contraste acquises à chaque intervalle de temps tₖ afin de générer n images d'échographie de contraste cumulatives (820 - 860, 920 - 960) ; et
un dispositif d'affichage (121, 122, 140, 440) conçu pour afficher successivement, dans l'ordre chronologique, les n images d'échographie de contraste cumulatives (820 - 860, 920 - 960) générées,
étant entendu que tₖ satisfait tₖ > tₖ₋₁.

2. Dispositif d'imagerie échographique (100, 100a, 100b, 100c, 300) selon la revendication 1, dans lequel la pression acoustique du signal ultrasonore (610, 620) est déterminée compte tenu du niveau d'énergie requis pour détruire l'agent de contraste.

3. Dispositif d'imagerie échographique (100, 100a, 100b, 100c, 300) selon la revendication 1, comprenant en outre une interface d'entrée (170) conçue pour recevoir une entrée utilisateur visant à définir tₖ et n,
le ou lesdits processeurs (120, 130, 320) étant conçus en outre pour définir tₖ et n compte tenu de l'entrée utilisateur.

4. Dispositif d'imagerie échographique (100, 100a, 100b, 100c, 300) selon la revendication 1, comprenant en outre une mémoire (150) dans laquelle sont stockées la pluralité d'images d'échographie de contraste acquises et la k^{ième} image d'échographie de contraste cumulative.

5. Dispositif d'imagerie échographique (100, 100a, 100b, 100c, 300) selon la revendication 1, dans lequel le ou les processeurs (120, 130, 320) sont conçus en outre pour corriger de flou de la pluralité d'images d'échographie de contraste dû aux mouvements de l'objet (10).

6. Dispositif d'imagerie échographique (100, 100a, 100b, 100c, 300) selon la revendication 1, dans lequel le ou les processeurs (120, 130, 320) sont conçus en outre pour générer une vidéo montée de telle manière que les n images d'échographie de contraste cumulatives (820 - 860, 920 - 960) sont diffusées successivement, et
le dispositif d'affichage (121, 122, 140, 330) est conçu en outre pour afficher la vidéo.

7. Dispositif d'imagerie échographique (100, 100a, 100b, 100c, 300) selon la revendication 1, dans lequel le ou les processeurs (120, 130, 320) sont conçus en outre pour générer des première à n^{ième} images d'échographie de contraste cumulatives (820 - 860, 920 - 960) totales en réalisant, n fois à partir de k=1 et jusqu'à k=n, un processus de génération d'une k^{ième} image d'échographie de contraste cumulative totale obtenue par cumulation des première à k^{ième} images d'échographie de contraste cumulatives (820 - 860, 920 - 960), et
le dispositif d'affichage (121, 122, 140, 330) est conçu en outre pour afficher les première à n^{ième} images d'échographie de contraste cumulatives (820 - 860, 920 - 960) totales.

8. Dispositif d'imagerie échographique (100, 100a, 100b, 100c, 300) selon la revendication 7, dans lequel
le ou les processeurs (120, 130, 320) sont conçus en outre pour générer une vidéo montée de telle manière que les première à n^{ième} images d'échographie de contraste cumulatives (820 - 860, 920 - 960) totales sont diffusées successivement, et
le dispositif d'affichage (121, 122, 140, 330) est conçu en outre pour afficher la vidéo.

9. Dispositif d'imagerie échographique (100, 100a, 100b, 100c, 300) comprenant :
une sonde (20, 310) conçue pour émettre un signal ultrasonore (610, 620) vers un objet (10) et pour détecter un signal d'écho ;
un ou plusieurs processeurs (120, 130, 320) conçus pour émettre un signal ultrasonore (610, 620), visant à détruire un agent de contraste, par la sonde (20, 310) vers une région d'intérêt de l'objet (10) dans laquelle l'agent de contraste a été injecté, pour acquérir fₖ images d'échographie de contraste de la région d'intérêt de l'objet (10) (k⊆1, 2, ..., n, et n est un entier valant deux ou plus), et pour générer n images d'échographie de contraste cumulatives en réalisant, n fois à partir de k=1 et jusqu'à k=n, un processus de génération d'une k^{ième} image d'échographie de contraste cumulative par cumulation des fₖ images d'échographie de contraste acquises ; et
un dispositif d'affichage (121, 122, 140, 330) conçu pour afficher successivement, dans l'ordre chronologique, les n images d'échographie de contraste cumulatives (820 - 860, 920 - 960) générées,
dans lequel fk satisfait fₖ > fₖ₋₁.

10. Dispositif d'imagerie échographique (100, 100a, 100b, 100c, 300) selon la revendication 9, dans lequel un ou plusieurs processeurs (120, 130, 320) sont conçus en outre pour générer une vidéo montée de telle manière que les n images d'échographie de contraste cumulatives sont diffusées successivement, et
le dispositif d'affichage (121, 122, 140, 330) est conçu en outre pour afficher la vidéo.

11. Dispositif d'imagerie échographique (100, 100a, 100b, 100c, 300) selon la revendication 9, dans lequel le ou les processeurs (120, 130, 320) sont conçus en outre pour générer des première à n^{ième} images d'échographie de contraste cumulatives totales en réalisant, n fois à partir de k=1 et jusqu'à k=n, un processus de génération d'une k^{ième} image d'échographie de contraste cumulative totale obtenue par cumulation des première à k^{ième} images d'échographie de contraste cumulatives, et
le dispositif d'affichage (121, 122, 140, 330) est conçu en outre pour afficher les première à n^{ième} images d'échographie de contraste cumulatives (820 - 860, 920 - 960) totales.

12. Procédé de commande d'un dispositif d'imagerie échographique (100, 100a, 100b, 100c, 300), le procédé comprenant :
l'émission d'un signal ultrasonore (610, 620), visant à détruire un agent de contraste, par une sonde (20, 310) vers une région d'intérêt de l'objet (10) dans laquelle l'agent de contraste a été injecté et l'acquisition d'une pluralité d'images d'échographie de contraste de la région d'intérêt de l'objet (10) à des intervalles de temps tₖ (k⊆1, 2, ..., n, et n est un entier valant deux ou plus), la génération d'une k^{ième} image d'échographie de contraste cumulative par cumulation de la pluralité d'images d'échographie de contraste acquises à chaque intervalle de temps tₖ afin de générer n images d'échographie de contraste cumulatives (820 - 860, 920 - 960) ; et
l'affichage successif, dans l'ordre chronologique, des n images d'échographie de contraste cumulatives (820 - 860, 920 - 960) générées,
étant entendu que tₖ satisfait tₖ > tₖ₋₁.

13. Procédé selon la revendication 12, comprenant en outre :
la génération d'une vidéo montée de telle manière que les n images d'échographie de contraste cumulatives sont diffusées successivement, et
l'affichage de la vidéo.

14. Procédé selon la revendication 12, comprenant en outre :
la génération de première à n^{ième} images d'échographie de contraste cumulatives totales en réalisant, n fois à partir de k=1 et jusqu'à k=n, un processus de génération d'une k^{ième} image d'échographie de contraste cumulative totale obtenue par cumulation des première à k^{ième} images d'échographie de contraste cumulatives, et
l'affichage des première à n^{ième} images d'échographie de contraste cumulatives totales.

15. Produit-programme informatique comprenant un support d'enregistrement lisible par ordinateur sur lequel sont stockées des instructions permettant de réaliser le procédé selon la revendication 12.
